# EUROPEAN PATENT APPLICATION

(11) **EP 4 467 658 A1**
(43) Date of publication of application: **27.11.2024**
(21) Application number: 23174843.5
(22) Date of filing: 23.05.2023
(51) Int. Cl.: C12Q 1/6804, C12Q 1/682, C12Q 1/6839

(54) **METHOD AND COMPACT MARKER FOR MARKING A BIOLOGICAL SAMPLE**

(71) Applicant: Leica Microsystems CMS GmbH, 35578 Wetzlar (DE); PixelBiotech GmbH, 69123 Heidelberg (DE)
(72) Inventor: Alsheimer, Soeren, 35578 Wetzlar (DE); Cheng, Yongsheng, 69123 Heidelberg (DE)
(74) Representative: Schaumburg und Partner Patentanwälte mbB

(57) **Abstract**

A method for marking a biological sample (300) is provided comprising the following steps: providing a biological sample (300) with at least one first target analyte (108); introducing into the biological sample (300) at least one first nucleic acid backbone (102), at least one first label configured to attach to the first nucleic acid backbone (102), and at least one first affinity reagent (106) specific to the first target analyte (108) and configured to attach to the first nucleic acid backbone (102); and introducing into the biological sample (300) a first plurality of staple strands (200), each staple strand (200) configured to hybridise to pairs of folding binding sites of the first nucleic acid backbone (102), in order to fold the first nucleic acid backbone (102) and generate a compact optically detectable first marker (202) in the biological sample (300). In a further aspect a compact marker (202) for marking a biological sample is provided.

## Description

### Technical field

The invention relates to a method and a compact marker for marking a biological sample including at least a first target analyte.

### Background

Biological samples, such as tissue sections, are often analysed after marking target analytes in the samples with makers specific to the target analytes. Such markers may comprise fluorophores that allow imaging the biological samples by means of a microscope in order to investigate the spatial distribution of the target analytes in the biological sample, for example. In order to be able to reliably detect these target analytes, in particular only rare target analytes, it is necessary to provide markers with optical properties that allow easy identification of these markers. For example, markers with fluorophores need to have a sufficiently bright fluorescent emission in order to reliably and routinely identify these markers in marker biological sample.

Often, markers with suitable optical properties, such as markers with several fluorophores come with trade-offs such as their large size that results in poor or insufficient penetration of the markers into the biological sample.

### Summary

It is an object to provide a marker and a method for marking biological sample that efficiently penetrate the biological sample and provide sufficient optical properties.

The aforementioned object is achieved by the subject-matter of the independent claims. Advantageous embodiments are defined in the dependent claims and the following description.

A method for marking a biological sample is provided comprising the following steps: providing a biological sample with at least one first target analyte; introducing into the biological sample at least one first nucleic acid backbone, at least one first label configured to attach to the first nucleic acid backbone, and at least one first affinity reagent specific to the first target analyte and configured to attach to the first nucleic acid backbone; and introducing into the biological sample a first plurality of staple strands, each staple strand configured to hybridise to pairs of folding binding sites of the first nucleic acid backbone, in order to fold the first nucleic acid backbone and generate a compact optically detectable first marker in the biological sample.

The introduction of the at least one first nucleic acid backbone, the at least one first label, and the at least one first affinity reagent separate from the introduction of the first plurality of staple strands enables generating the compact first marker within the biological sample rather than outside of the biological sample. Since the first nucleic acid backbone and the staple strands separately are small and/or have a high degree of freedom in their movement and bending, they easily and efficiently penetrate the biological sample. For example, the staple strands are preferably 15 to 60 nucleotides in length. The compact marker instead, is bulky and does not efficiently penetrate the biological sample. Since the compact marker is generated in situ, the target analyte may still be efficiently marked by means of the compact marker.

Preferably, the compact marker is formed by one first nucleic acid backbone, one first label, and one first affinity reagent. The first label and the first affinity reagent may comprise oligomers that have a unique sequence at least partly complementary to a respective sequence of the nucleic acid backbone for attaching the first label and the first affinity reagent to a predetermined location on the nucleic acid backbone.

Preferably, the step b) and step c) are carried out subsequently. Thus, the steps b) and c) are preferably not carried out simultaneously, but either step c) follows step b) or step b) follows step c). This enables generating the marker particularly efficiently within the biological sample after the at least one first nucleic acid backbone, the at least one first label, the at least one first affinity reagent, and/or the first plurality of staple strands of step b) and/or step c) have penetrated the biological sample.

Preferably, a step d) of waiting for a time in a range of 10 s to 120 min; preferably 5 min to 90 min, more preferably 10 min to 60 min is carried out between steps b) and c). This enables ensuring that the at least one first nucleic acid backbone, the at least one first label, the at least one first affinity reagent, and/or the first plurality of staple strands of step b) and/or step c). In particular, this time may depend on a temperature of the biological sample, for example, a time of 5 min at 45°C to 60°C, whereas a time of 10 min to 60 min may be waited at 37°C. In certain cases, the waiting time may be significantly longer up to multiple days, when, for example, entire mouse embryos or large pieces of tissue shall be stained. In such cases the method described in this document may be combined with organic solvent- or water-based clearing methods like for example Spalteholz clearing and related technique, CLARITY, Scale, Cubic, DISCO, iDISCO and similar methods. This may further aid penetration of the biological sample by the at least one first nucleic acid backbone, the at least one first label, the at least one first affinity reagent, and/or the first plurality of staple strands.

Preferably, in step d) additionally an electrophoresis electric field is applied to the biological sample. This may enable or assist particularly efficient introduction of the at least one first nucleic acid backbone, the at least one first label, the at least one first affinity reagent, and/or the first plurality of staple strands into the biological sample. This field may have a stable or alternating directionality.

Preferably, the first nucleic acid backbone, the first label and the first affinity reagent of step b) are introduced into a first region of the biological sample and the first plurality of staple strands of step c) are introduced into a second region of the biological sample, wherein the first region and the second region are spatially apart from each other. Particularly, in this case, the at least one first nucleic acid backbone, the at least one first label, the at least one first affinity reagent, and/or the first plurality of staple strands may be introduced into the biological sample at the same time. This enables particularly efficient introduction of the at least one first nucleic acid backbone, the at least one first label, the at least one first affinity reagent, and/or the first plurality of staple strands into the biological sample. For example, in case the biological sample is a planar tissue section, the at least one first nucleic acid backbone, the at least one first label, and the at least one first affinity reagent may be introduced into the biological sample from one side of the plane of the tissue section and the first plurality of staple strands may be introduced into the biological sample from the other side of the plane of the tissue section.

According to a prefered embodiment, a step e), preferably carried out prior to steps b) and c), comprises adjusting at least one environmental parameter of the biological sample to a first condition under which the first plurality of staple strands predominantly do not hybridise to the folding binding sites of the first nucleic acid backbone and wherein a step f), preferably carried out after steps b) and c), comprises adjusting the at least one environmental parameter of the biological sample to a second condition under which the first plurality of staple strands predominantly do hybridise to the folding binding sites of the first nucleic acid backbone. The environmental parameter may be a buffer salt type, a buffer salt concentration, or a temperature. Predominantly in this context means, that more than 50%, preferably more than 70%, more preferably more than 80%, of the staple strands are not or are hybridised to the nucleic acid backbone, respectively. Thus, the change in the environmental parameter from the first condition to the second condition, for example by a buffer exchange or a temperature shift, influences a binding coefficient and may trigger the staple strand hybridisation and the folding of the compact marker.

Preferably, the first affinity reagent is an oligonucleotide, an antibody, or an antibody fragment. This enables binding of the marker with high specificity to the target analyte. Particularly preferred is an aptamer. This enables generating particularly small and compact markers with high specificity to the target analyte.

Preferably, a largest spatial extent of the first affinity reagent is in a range of 1 to 25 nm, more preferably in a range of 1 to 10 nm. Alternatively, the first affinity reagent is preferably in a range of 5 to 200 kDa, more preferably 5 to 70 kDa, even more preferably 5 to 30 kDa. This enables generating particularly small and compact markers with high specificity to the target analyte. Antibody fragments, single-domain antibodies, nanobodies, and aptamers are particularly suited first affinity reagents. Alternatively, or in addition oligonucleotide probes may serve as first affinity reagents to label nucleic acid targets such as genomic DNA or mRNA transcripts.

Preferably, the first nucleic acid backbone has a length in a range of 100 to 3000 nm, preferably in a range of 500 to 2500 nm. For example, the nucleic acid backbone is generated from a M13 bacteriophage genome of approximately 7000 nucleotides in size. This enables generating a marker with particularly bright label, for example, by enabling attachment of a large number of optically detectable moieties.

Preferably, the first nucleic acid backbone of the first marker is folded into a shape that fits in a cuboid with side lengths in a range of 10 to 1000 nm, preferably 50 to 200 nm. This enables generating a particularly compact marker.

Preferably, the biological sample is a section, for example, a tissue section, or a microtome or vibratome section, with a thickness in a range of 1 to 200 µm, preferably 5 nm to 100 µm. The method enables penetrating sections of thickness up to 200 µm.

Preferably, the first label comprises 1 to 200, preferably 50 to 100, optically detectable moieties. Each label may comprise at least one fluorophore, polyyne or other optically detectable moiety. This enables generating particularly bright markers.

Preferably, in step b) at least one second nucleic acid backbone, at least one second label configured to attach to the second nucleic acid backbone, and at least one second affinity reagent specific to a second target analyte in the biological sample and configured to attach to the second nucleic acid backbone is introduced into the biological sample, and wherein in step c) a second plurality of staple strands is introduced into the biological sample, each staple strand configured to hybridise to pairs of folding binding sites of the second nucleic acid backbone in order to fold the second nucleic acid backbone and generate a compact optically detectable second marker in the biological sample. This enables particularly efficient marking of several target analytes in the biological sample. The introduction of the components in each step mentioned above may be done essentially simultaneously or subsequently, either at the same or at different regions of the biological sample.

In another aspect, a nucleic acid backbone construct is provided, comprising: a nucleic acid backbone with pairs of folding binding sites configured to hybridise to complementary pairs of folding binding sites of staple strands; a label attached to the nucleic acid backbone, and an affinity reagent specific to a target analyte and bound to the nucleic acid backbone. Preferably, the nucleic acid backbone construct is configured such that the method according to one of the claims 1 to 13 can be carried out.

In yet another aspect, a marker for marking a biological sample is provided, comprising: the nucleic acid backbone construct; and staple strands comprising pairs of binding sites hybridised to the complementary pairs of folding binding sites of the nucleic acid backbone.

### Short Description of the Figures

Hereinafter, specific embodiments are described referring to the drawings, wherein:
- Figure 1: is a schematic view of a nucleic acid construct for a marker,
- Figure 2: is a schematic view of the nucleic acid construct according to Fig. 1 with staple strands forming a marker, and
- Figure 3: is a flow diagram of a method for marking a biological sample with the marker according to Fig. 2.

### Detailed Description

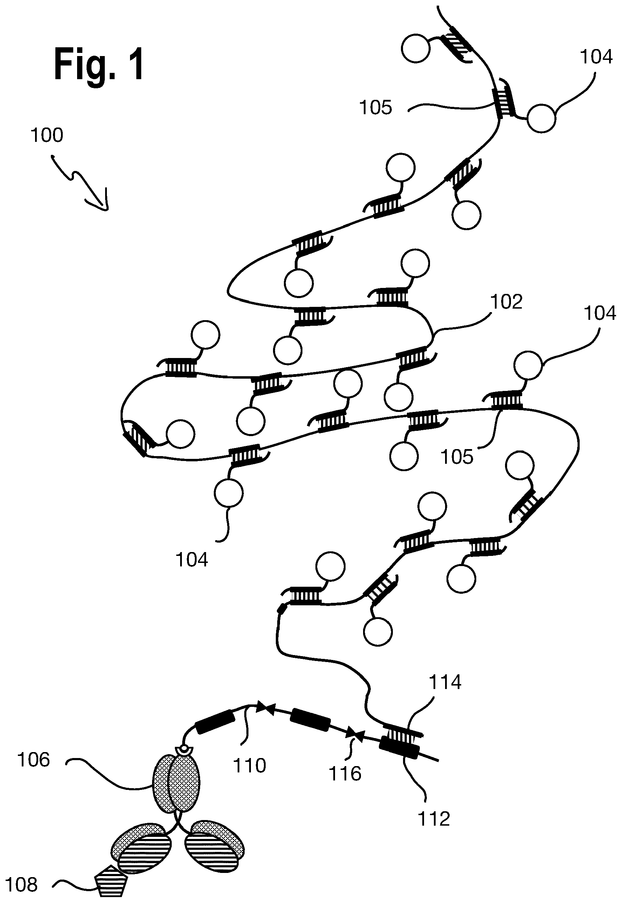

Figure 1 is a schematic view of a nucleic acid construct 100. The nucleic acid construct 100 may be used to generate a marker, as is explained subsequently. The nucleic acid construct 100 comprises a nucleic acid backbone 102. The nucleic acid backbone 102 preferably comprises 300 to 10000 nucleotides. Thus, it is preferably 100 to 3000 nm long. For example, the nucleic acid backbone 102 may comprise bacteriophage-derived genomic DNA manufactured using high cell-density fermentation of E. coli in stirred-tank bioreactors. In particular, the nucleic acid backbone 102 may be generated from M13 bacteriophage and comprise approximately 7000 nucleotides.

Attached to the nucleic acid backbone 102 is a label comprising a plurality of optically detectable moieties 104. The optically detectable moieties 104 may be fluorophores with the same optical properties such as the same excitation and emission wavelength, for example. Alternatively, the optically detectable moieties 104 may have a variety of different optical properties. The optically detectable moieties 104 may be individually attached to the nucleic acid backbone 102 at specific binding sites 105 along the nucleic acid backbone 102.

Additionally, an affinity reagent 106 is attached to the nucleic acid backbone 102. The affinity reagent 106 may be an antibody, for example. Preferably, the affinity reagent 106 is smaller than an antibody, for example, the affinity reagent 106 may be an antibody fragment, in particular a Fab fragment, nanobody or an aptamer. Preferably, the affinity reagent 106 has a size in the range of 1 to 10 nm, or alternatively in a range of 5 to 30 kDa.

The affinity reagent 106 is specific to a target analyte 108 in a biological sample and therefore is configured to bind the nucleic acid construct 100 specifically to the target analyte 108. The affinity reagent 106 may comprise an oligomer 110 that comprises a binding site 112 that specifically binds to a complementary binding site 114 of the nucleic acid backbone 102. Further, the oligomer 110 may comprise one or more cleavage sites 116, for example a restriction site, that allow cleaving the oligomer 110 and thereby removing the nucleic acid backbone 102 with the optically detectable moieties 104 from the affinity reagent 106. This is particularly useful when iteratively staining the biological sample.

Alternatively, the nucleic acid backbone 102 may be directly attached to the affinity reagent 106, without the oligomer 110.

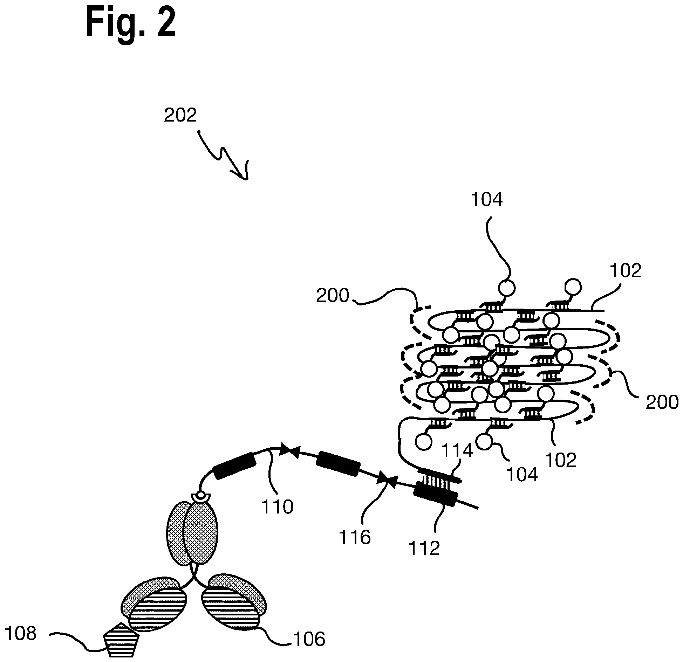

Figure 2 is a schematic view of the nucleic acid construct 100 according to Figure 1 and staple strands 200 that form a marker 202 together with the nucleic acid construct 100. The nucleic acid backbone 102 comprises pairs of folding binding sites that the staple strands 200 are configured to bind to. The staple strands 200 are nucleic acid strands. Thus, each of the staple strands 200 has complementary binding sites that bind to at least one of the pairs of folding binding sites of the nucleic acid backbone 102. There may be a plurality of staple strands 200 that each specifically bind to a particular pair of complementary and unique folding binding sites on the nucleic acid backbone 102. The pairs of folding binding sites are arranged on the nucleic acid backbone 102 such that when the staple strands 200 bind to the folding binding sites of the nucleic acid backbone 102, the nucleic acid backbone 102 folds into a compact structure and thus generates the compact optically detectable marker 202. For example, the linear nucleic acid backbone 102 may be folded into a sheet as shown in Figure 2 by pulling and binding together the linear strand of the nucleic acid backbone 102 at regular intervals with the staple strands 200. Alternative geometrical shapes of a compact marker 202 might be generated depending on the specific configuration and characteristics of the staple strands 200, e.g. spheric, cubic, tetrahedral, or polyhedral shapes.

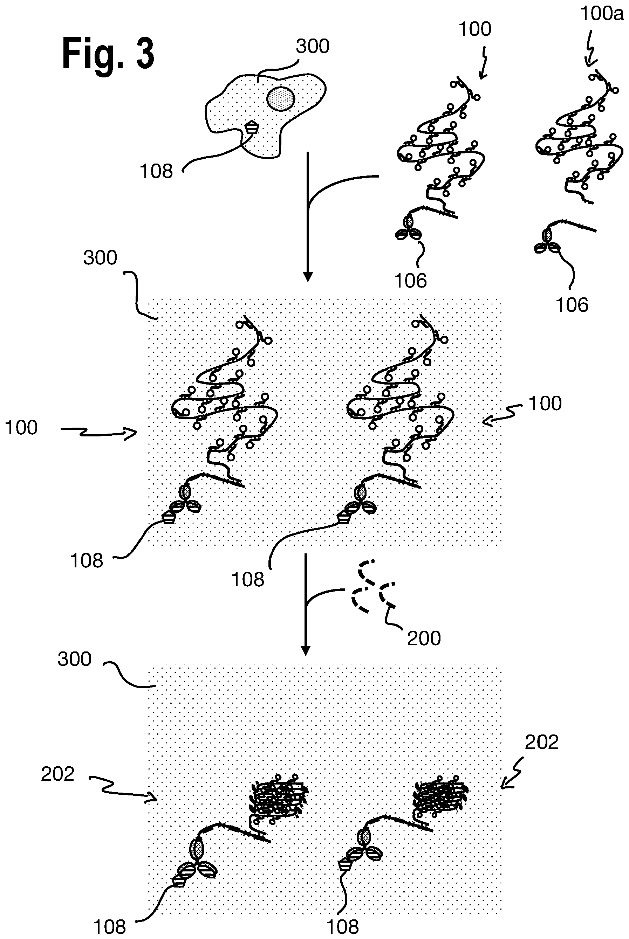

Figure 3 is schematic representation of a flow diagram of a method for marking a biological sample 300 with the marker 202 according to Figure 2. The biological sample 300 comprises at least the target analyte 108 and the method initially starts with providing the biological sample 300 with the target analyte 108. The biological sample 300 may be a tissue section, in particular an expanded tissues section, for example.

Further, the nucleic acid backbone 102, the label comprising the optically detectable moieties 104 and the affinity reagent 106 of the nucleic acid construct 100 are introduced into the biological sample 300. Generally, several copies of the nucleic acid backbone 102, the label comprising the optically detectable moieties 104 and the affinity reagent 106 are introduced, with one nucleic acid backbone 102, one label comprising the optically detectable moieties 104 and one affinity reagent 106 forming one nucleic acid construct 100.

The nucleic acid construct 100 may have already been formed prior to introducing the construct 100 into the biological sample 300. Alternatively, the individual nucleic acid backbones 102, the labels and the affinity reagents 106 may be introduced into the biological sample 300. The nucleic acid construct 100 then forms within the biological sample 300. For example, a partial nucleic acid construct 100a comprising the nucleic acid backbone 102 and bound label 104 may be introduced separately from the affinity reagent 106.

Within the biological sample 300, the nucleic acid construct 100 binds to the target analyte 108. Subsequently, the staple strands 200 may be introduced into the biological sample 300 in order to generate the compact marker 202 within the biological sample 300.

Alternatively, the staple strands 200 may be introduced into the biological sample 300 first and subsequently the nucleic acid construct 100 may be introduced. In a further alternative, the nucleic acid construct 100 and the staple strands 200 may be introduced at the same time, however, at different regions of the biological sample 300. For example, in case the biological sample 300 is a planar tissue section the nucleic acid construct 100 may be introduced into the biological sample 300 on one side of the plane of the tissue section and the staple strands 200 may be introduced into the biological sample 300 on the opposite side of the plane of the tissue section.

The nucleic acid construct 100 and the staple strands 200 generally penetrate the biological sample 300 by diffusion. Further, the individual components, in particular the nucleic acid construct 100 and the staple strands 200, generally penetrate the biological sample 300 at a faster rate than the compact marker 202. This is due to the bulk and inflexibility of the compact marker 202 in comparison to the linear shape of the nucleic acid construct 100 and the high degree of freedom of its nucleic acid backbone 102, when no staple strands 200 are bound to it. In order to predominantly generate the compact marker within the biological sample 300, it is preferred to introduce the staple strands 200 and the nucleic acid construct 100 into the biological sample 300 separately. Once the staple strands 200 and the nucleic acid construct 100 are diffused into the biological sample 300 and are in the same area within the sample 300, the compact marker 202 is formed. The compact marker 202 is advantageous since it reduces the area over which the label, in particular the optically detectable moieties 104, are distributed and therefore provides a marker that provides particularly bright spot at or below the diffraction limit of an optical imaging system, e.g. a light microscope, used to image the marked biological sample 300.

Thus, the nucleic acid construct 100 and the staple strands 200 are preferably separately introduced into the biological sample 300, separated either temporally (subsequently to each other) and/or spatially (in different regions of the biological sample 300), as described above.

In addition to diffusion, the nucleic acid construct 100 and/or the staple strands 200 may be actively introduced into the sample 300 by means of electrophoresis by applying an electric field to the sample 300. Such an electric field can be altered or modulated, e.g. with regard to the magnitude of the electric field and/or the direction distribution of the vector field of the electric field, which might support diffusion of the nucleic acid construct 100 and/or the staple strands 200 in the sample 300.

Further, environmental parameters of the biological sample 300 may be adjusted in order to suppress or promote the hybridisation of the staple strands 200 with the nucleic acid backbone 102 and the generation of the compact marker 202. For example, before introducing the nucleic acid construct 100 and the staple strands 200 into the biological sample 300, the salt and/or concentration of a buffer the biological sample 300 is in may be chosen to suppress binding of the staple strands 200 to the nucleic acid backbone 102. After introduction of the nucleic acid construct 100 and the staple strands 200 into the biological sample 300, the salt and/or concentration of a buffer the biological sample 300 is in may be changed to promote binding of the staple strands 200 to the nucleic acid backbone 102. Alternatively or in addition, the temperature the sample 300 is at may be adjusted in order to suppress or promote binding of the staple strands 200 to the nucleic acid backbone 102.

With the steps described above for generating the marker 202 within the biological sample 300, further markers may be generated simultaneously. For example, similarly to the steps described above, a second nucleic acid backbone, a second label configured to bind to the second nucleic acid backbone and a second affinity reagent configured to bind to the second nucleic acid backbone may be introduced into the biological sample 300. A second marker may be generated by introducing second staple strands configured to bind to pairs of folding binding sites of the second nucleic acid backbone in order to generate the second marker.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100: Nucleic acid construct
- 100a: Partial nucleic acid construct
- 102: Nucleic acid backbone
- 104: Optically detectable moiety
- 105: Binding site of optically detectable moiety
- 106: Affinity reagent
- 108: Target analyte
- 110: Oligomer
- 112: Binding site on oligomer
- 114: Binding site on backbone
- 116: Cleavage site
- 200: Staple strand
- 202: Compact marker
- 300: Biological sample

## Claims

1. A method for marking a biological sample (300),
a) providing a biological sample (300) with at least one first target analyte (108),
b) introducing into the biological sample (300) at least one first nucleic acid backbone (102), at least one first label configured to attach to the first nucleic acid backbone (102), and at least one first affinity reagent (106) specific to the first target analyte (108) and configured to attach to the first nucleic acid backbone (102), and
c) introducing into the biological sample (300) a first plurality of staple strands (200) each staple strand (200) configured to hybridise to pairs of folding binding sites of the first nucleic acid backbone (102) in order to fold the first nucleic acid backbone (102) and generate a compact optically detectable first marker (202) in the biological sample (300).

2. The method according to claim 1, wherein step b) and step c) are carried out subsequently.

3. The method according to one of the preceding claims, wherein a step d) of waiting for a time in a range of 10 s to 120 min; preferably 5 min to 90 min, more preferably 10 min to 60 min is carried out between steps b) and c).

4. The method according to claim 3, wherein in step d) additionally an electrophoresis electric field is applied to the biological sample.

5. The method according to one of the preceding claims, wherein the first nucleic acid backbone (102), the first label and the first affinity reagent (106) of step b) are introduced into a first region of the biological sample (300) and the first plurality of staple strands (200) of step c) are introduced into a second region of the biological sample (300), wherein the first region and the second region are spatially apart from each other.

6. The method according to one of the preceding claims, wherein a step e), preferably carried out prior to steps b) and c), comprises adjusting at least one environmental parameter of the biological sample (300) to a first condition under which the first plurality of staple strands (200) predominantly do not hybridise to the folding binding sites of the first nucleic acid backbone (102) and wherein a step f), preferably carried out after steps b) and c), comprises adjusting the at least one environmental parameter of the biological sample to a second condition under which the first plurality of staple strands (200) predominantly do hybridise to the folding binding sites of the first nucleic acid backbone (102).

7. The method according to one of the preceding claims, wherein the first affinity reagent (106) is an oligonucleotide, an antibody, or an antibody fragment.

8. The method according to one of the preceding claims, wherein a largest spatial extent of the first affinity reagent (106) is in a range of 1 to 25 nm.

9. The method according to one of the preceding claims, wherein the first nucleic acid backbone (102) has a length in a range of 100 to 3000 nm, preferably in a range of 500 to 2500 nm.

10. The method according to one of the preceding claims, wherein the first nucleic acid backbone (102) of the first marker (202) is folded into a shape that fits in a cuboid with side lengths in a range of 10 to 1000 nm, preferably 50 to 200 nm.

11. The method according to one of the preceding claims, wherein the biological sample (300) is a section with a thickness in a range of 1 to 200 µm, preferably 5 nm to 100 µm.

12. The method according to one of the preceding claims, wherein the first label comprises 1 to 200, preferably 50 to 100, optically detectable moieties (104).

13. The method according to one of the preceding claims, wherein in step b) at least one second nucleic acid backbone, at least one second label configured to attach to the second nucleic acid backbone, and at least one second affinity reagent specific to a second target analyte in the biological sample and configured to attach to the second nucleic acid backbone is introduced into the biological sample, and wherein in step c) a second plurality of staple strands is introduced into the biological sample, each staple strand configured to hybridise to pairs of folding binding sites of the second nucleic acid backbone in order to fold the second nucleic acid backbone and generate a compact optically detectable second marker in the biological sample.

14. A nucleic acid backbone construct (100) comprising:
a nucleic acid backbone (102) with pairs of folding binding sites configured to hybridise to complementary pairs of folding binding sites of staple strands (200),
a label attached to the nucleic acid backbone (102), and
an affinity reagent (106) specific to a target analyte (108) and bound to the nucleic acid backbone (102).

15. A marker (202) for marking a biological sample (300) comprising:
the nucleic acid backbone construct (100) according to claim 14, and
staple strands (200) comprising pairs of binding sites hybridised to the complementary pairs of folding binding sites of the nucleic acid backbone (102).
